# EUROPEAN PATENT APPLICATION

(11) **EP 4 398 264 A1**
(43) Date of publication of application: **10.07.2024**
(21) Application number: 22864779.8
(22) Date of filing: 10.02.2022
(51) Int. Cl.: G16H 50/50, G16H 50/70, G16H 30/40, G16H 50/20, G06N 3/04, G06N 3/08

(54) **DEEP LEARNING-BASED METHOD AND DEVICE FOR PREDICTING ANALYSIS RESULTS**

(30) Priority: 02.09.2021 KR 20210117172
(71) Applicant: Kwangwoon University Industry-Academic Collaboration Foundation, Seoul 01897 (KR)
(72) Inventor: LEE, Jeong Hoon, Seongnam-si Gyeonggi-do 13646 (KR); LEE, Ki Baek, Namyangju-si Gyeonggi-do 12098 (KR); YOO, Yong Kyoung, Seoul 02063 (KR); LEE, Seung Min, Seoul 02230 (KR); LEE, Hak Jun, Seoul 04957 (KR); MOON, Ji Won, Seoul 06984 (KR)
(74) Representative: Ghirardi, Valeria
(86) International application number: PCT/KR2022/002020
(87) International publication number: WO 2023/033270

(57) **Abstract**

According to a preferred embodiment of the present invention, a deep learning-based method and device for predicting analysis results predict analysis results of immune response assay-based kits, such as that of lateral flow assay (LFA), antigen-antibody-based diagnostic kits and the like on the basis of deep learning, and thus can reduce the time for confirming results.

## Description

### [Technical Field]

The present invention relates to a deep learning-based method and a device for predicting analysis results, and more particularly, to a method and a device for predicting analysis results based on deep learning.

### [Background Art]

It takes a long time (10 to 30 minutes) for a diagnostic test through a lateral flow assay (LFA) reaction which collects samples from a specimen and uses the collected samples.. The lateral flow assay shows different aspects depending on a sample concentration and a reaction time and the diagnostic test using the lateral flow assay (LFA) reaction can only be determined when a sufficient reaction occurs after approximately 15 minutes. However, in the case of certain diseases, such as myocardial infarction, results are frequently requested within 10 minutes and recently, the need for a quick diagnosis within five minutes has increased significantly from the perspective of hospitals and patients.

### [Disclosure]

### [Technical Problem]

An object to be achieved by the present invention is to provide a deep learning-based analysis result predicting method and device which predict analysis results of immune response assay-based kit such as a lateral flow assay (LFA) and antigen-antibody-based diagnostic kits on the basis of deep learning.

Other and further objects of the present invention which are not specifically described can be further considered within the scope easily deduced from the following detailed description and the effect.

### [Technical Solution]

In order to achieve the above-described object, according to a preferred embodiment of the present invention, a deep learning-based analysis result predicting method includes a step of obtaining a reaction image for a predetermined initial period for an interaction of a sample obtained from a specimen and an optical-based kit; and a step of predicting a concentration for a predetermined result time on the basis of the reaction image for the predetermined initial period, using a pre-trained and established analysis result prediction model.

Here, the step of obtaining a reaction image is configured by obtaining a plurality of reaction images in a predetermined time unit for the predetermined initial period.

Here, the analysis result prediction model includes: an image generator includes a convolution neural network (CNN), long short-term memory (LSTM), and a generative adversarial network (GAN), and generates a prediction image corresponding to a predetermined result time on the basis of an input reaction image, and outputs the generated prediction image; and a regression model which includes the convolution neural network (CNN) and outputs a predicted concentration for a predetermined result time on the basis of the prediction image generated by the image generator, and the regression model is trained using the learning data to minimize the difference of the predicted concentration for the predetermined result time obtained on the basis of the reaction image of the learning data and the actual concentration for the predetermined result time of the learning data.

Here, the image generator includes: an encoder which obtains a feature vector from the input reaction image using the convolution neural network (CNN), obtains a latent vector on the basis of the obtained feature vector using the long short term memory (LSTM), and outputs the obtained latent vector; and a decoder which generates the prediction image on the basis of the latent vector obtained from the encoder using the generative adversarial network (GAN), and outputs the generated prediction image.

Here, the decoder includes a generator which generates the prediction image on the basis of the latent vector and outputs the generated prediction image; and a discriminator which compares the prediction image generated by the generator and an actual image corresponding to a predetermined result time of the learning data and outputs a comparison result, and it is trained to discriminate that the prediction image obtained on the basis of the latent vector is the actual image using the learning data.

Here, the step of obtaining a reaction image is configured by obtaining the reaction image of an area corresponding to the test-line when the optical-based kit includes a test-line and a control-line.

Here, the step of obtaining a reaction image is configured by obtaining the reaction image of the area corresponding to one or more predetermined test-lines, among a plurality of test-lines when the optical-based kit includes a plurality of test-lines.

Here, the step of obtaining a reaction image is configured by obtaining the reaction image including all areas corresponding to one or more predetermined test-lines, among the plurality of test-lines or obtaining the reaction image for every test-line to distinguish areas corresponding to one or more predetermined test-lines, among the plurality of test-lines for every test-line.

In order to achieve the above-described technical object, according to a preferred embodiment of the present invention, a computer program is stored in a computer readable storage medium to allow a computer to execute any one of the deep learning-based analysis result predicting methods.

In order to achieve the above-described object, according to a preferred embodiment of the present invention, a deep learning-based analysis result predicting device is a deep learning-based analysis result predicting device which predicts an analysis result based on deep learning and includes a memory which stores one or more programs to predict an analysis result; and one or more processors which perform an operation for predicting the analysis result according to one or more programs stored in the memory, and the processor predicts a concentration for a predetermined result time on the basis of a reaction image of a predetermined initial period for an interaction of a sample obtained from a specimen and an optical-based kit, using a pre-trained and established analysis result prediction model.

Here, the processor obtains a plurality of reaction images in a predetermined time unit for the predetermined initial period.

Here, the analysis result prediction model includes: an image generator includes a convolution neural network (CNN), long short-term memory (LSTM), and a generative adversarial network (GAN), and generates a prediction image corresponding to a predetermined result time on the basis of an input reaction image, and outputs the generated prediction image; and a regression model which includes the convolution neural network (CNN) and outputs a predicted concentration for a predetermined result time on the basis of the prediction image generated by the image generator, and the regression model is trained using the learning data to minimize the difference of the predicted concentration for the predetermined result time obtained on the basis of the reaction image of the learning data and the actual concentration for the predetermined result time of the learning data.

Here, the image generator includes: an encoder which obtains a feature vector from the input reaction image using the convolution neural network (CNN), obtains a latent vector on the basis of the obtained feature vector using the long short term memory (LSTM), and outputs the obtained latent vector; and a decoder which generates the prediction image on the basis of the latent vector obtained from the encoder using the generative adversarial network (GAN), and outputs the generated prediction image.

### [Advantageous Effects]

According to the deep learning-based analysis result predicting method and device according to a preferred embodiment of the present invention, analysis results of immune response assay-based kit such as a lateral flow assay (LFA) and antigen-antibody-based diagnostic kits are predicted on the basis of deep learning to reduce the time for confirming results.

The effects of the present disclosure are not limited to the technical effects mentioned above, and other effects which are not mentioned can be clearly understood by those skilled in the art from the following description

### [Brief Description of Drawings]

FIG. 1 is a block diagram for explaining a deep learning-based analysis result predicting device according to a preferred embodiment of the present invention.
FIG. 2 is a view for explaining a process of predicting analysis results according to a preferred embodiment of the present invention.
FIG. 3 is a view for explaining a process of predicting changes of color intensity over time based on a reaction image according to a preferred embodiment of the present invention.
FIG. 4 is a view for explaining a process of predicting a concentration for a result time on the basis of a reaction image for an initial period according to a preferred embodiment of the present invention.
FIG. 5 is a flowchart for explaining a deep learning-based analysis result predicting method according to a preferred embodiment of the present invention.
FIG. 6 is a view for explaining an example of a structure of an analysis result prediction model according to a preferred embodiment of the present invention.
FIG. 7 is a view for explaining an implementation example of an analysis result prediction model illustrated in FIG. 6.
FIG. 8 is a view for explaining another example of a structure of an analysis result prediction model according to a preferred embodiment of the present invention.
FIG. 9 is a view for explaining learning data used for a learning process of an analysis result prediction model according to a preferred embodiment of the present invention.
FIG. 10 is a view for explaining a configuration of learning data illustrated in FIG. 9.
FIG. 11 is a view for explaining an example of a reaction image illustrated in FIG. 10.
FIG. 12 is a view for explaining an example of a pre-processing process of a reaction model according to a preferred embodiment of the present invention.
FIG. 13 illustrates three representative commercialized diagnostic tools.
FIG. 14 illustrates an example of a deep learning architecture,
FIG. 15 illustrates an assessment of infectious diseases, specifically COVID-19 antigen and Influenza A/B, using a 2-minute assay facilitated by the TIMESAVER model.
FIG. 16 illustrates an assessment of non-infectious biomarkers for emergency room (ER) via the TIMESAVER model.
FIG. 17 illustrates the clinical evaluation of COVID-19 through blind tests.

### [Mode for carrying out the disclosure]

Hereinafter, embodiments of the present invention will be described in detail with reference to the accompanying drawings. Advantages and characteristics of the present invention and a method of achieving the advantages and characteristics will be clear by referring to preferable embodiments described below in detail together with the accompanying drawings. However, the present invention is not limited to preferable embodiments disclosed herein, but will be implemented in various different forms. The preferable embodiments are provided by way of example only so that a person of ordinary skilled in the art can fully understand the disclosures of the present invention and the scope of the present invention. Therefore, the present invention will be defined only by the scope of the appended claims. Like reference numerals generally denote like elements throughout the specification.

Unless otherwise defined, all terms (including technical and scientific terms) used in the present specification may be used as the meaning which may be commonly understood by the person with ordinary skill in the art, to which the present disclosure belongs. It will be further understood that terms defined in commonly used dictionaries should not be interpreted in an idealized or excessive sense unless expressly and specifically defined.

In the specification, the terms "first" and "second" are used to distinguish one component from the other component so that the scope should not be limited by these terms. For example, a first component may also be referred to as a second component and likewise, the second component may also be referred to as the first component.

In the present specification, in each step, numerical symbols (for example, a, b, and c) are used for the convenience of description, but do not explain the order of the steps so that unless the context apparently indicates a specific order, the order may be different from the order described in the specification. In the present specification, in each step, numerical symbols (for example, a, b, and c) are used for the convenience of description, but do not explain the order of the steps so that unless the context apparently indicates a specific order, the order may be different from the order described in the specification.
In this specification, the terms "have", "may have", "include", or "may include" represent the presence of the characteristic (for example, a numerical value, a function, an operation, or a component such as a part"), but do not exclude the presence of additional characteristic.

Hereinafter, a preferred embodiment of a deep learning based analysis result predicting method and device according to the present invention will be described in detail with reference to the accompanying drawings.

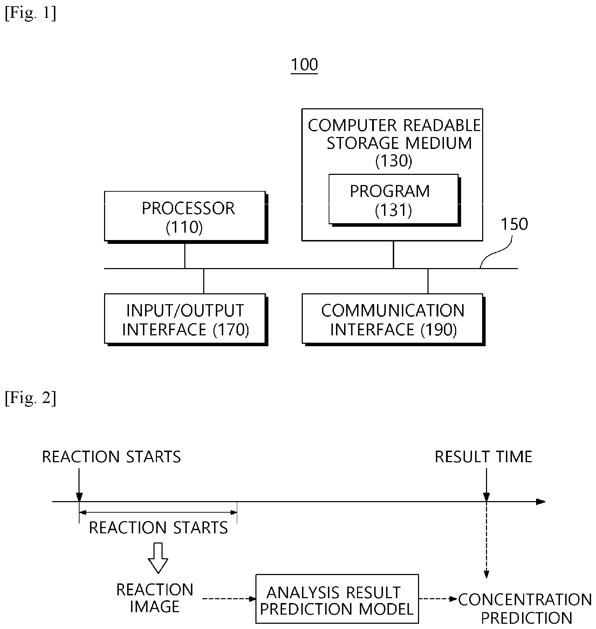

First, a deep learning based analysis result predicting device according to the present invention will be described with reference to FIGS. 1 to 4.

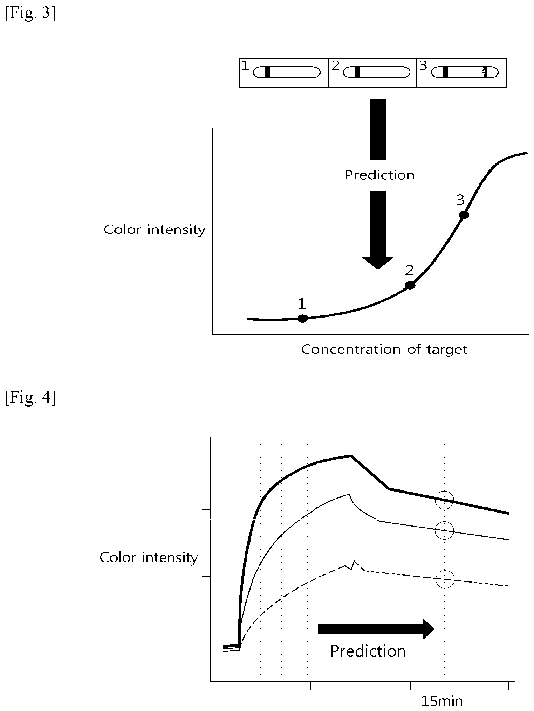

FIG. 1 is a block diagram for explaining a deep learning-based analysis result predicting device according to a preferred embodiment of the present invention, FIG. 2 is a view for explaining a process of predicting analysis results according to a preferred embodiment of the present invention, FIG. 3 is a view for explaining a process of predicting changes of color intensity over time based on a reaction image according to a preferred embodiment of the present invention, and FIG. 4 is a view for explaining a process of predicting a concentration for a result time on the basis of a reaction image for an initial period according to a preferred embodiment of the present invention.

Referring to FIG. 1, a deep learning-based analysis result predicting device according to a preferred embodiment of the present invention (hereinafter, referred to as an "analysis result predicting device) 100 predicts analysis results of immune response assay-based kits such as lateral flow assay (LFA) and antigen-antibody-based diagnostic kits on the basis of deep learning.

In the meantime, an operation of predicting analysis results on the basis of deep learning according to the present invention may be applied not only to lateral flow assay which derives a result on the basis of an color intensity, but also to another analysis which derives a result on the basis of fluorescence intensity. However, for the convenience of description of the present invention, the following description will be made under the assumption that the present invention predicts lateral flow assay results.

To this end, the analysis result predicting device100 may include one or more processors 110, a computer readable storage medium 130, and a communication bus 150.

The processor 110 controls the analysis result predicting device 100 to operate. For example, the processor 110 may execute one or more programs 131 stored in the computer readable storage medium 130. One or more programs 131 include one or more computer executable instructions and when the computer executable instruction is executed by the processor 110, the computer executable instruction may be configured to allow the analysis result predicting device 100 to perform an operation for predicting a result of analysis (for example, lateral flow assay).

The computer readable storage medium 130 is configured to store a computer executable instruction or program code, program data and/or other appropriate format of information to predict a result of analysis (for example, lateral flow assay). The program 131 stored in the computer readable storage medium 130 includes a set of instructions executable by the processor 110. In one preferable embodiment, the computer readable storage medium 130 may be a memory (a volatile memory such as a random access memory, a non-volatile memory, or an appropriate combination thereof), one or more magnetic disk storage devices, optical disk storage devices, flash memory devices, and another format of storage media which are accessed by the analysis result predicting device 100 and store desired information, or an appropriate combination thereof.

The communication bus 150 interconnects various other components of the analysis result predicting device 100 including the processor 110 and the computer readable storage medium 130 to each other.

The analysis result predicting device 100 may include one or more input/output interfaces 170 and one or more communication interfaces 190 which provide an interface for one or more input/output devices. The input/output interface 170 and the communication interface 190 are connected to the communication bus 150. The input/output device (not illustrated) may be connected to another components of the analysis result predicting device 100 by means of the input/output interface 170.

Referring to FIG. 2, the processor 110 of the analysis result predicting device 100 predicts a concentration for a predetermined result time based on a reaction image of a predetermined initial period for interaction of samples obtained from specimen and optical-based kits (for example, immune response assay-based kits such as lateral flow assay (LFA), or an antigen-antibody-based diagnostic kits, such as LSPR, SPR, and fluorescence based assay) using an analysis result prediction model which is pre-trained and established.

Here, the predetermined initial period and the predetermined result time may vary depending on a kind or a type of the optical based kit and specifically, the predetermined result time refers to a time that a final result for the sample is confirmed. For example, the predetermined result time may be set to "15 minutes" and the predetermined initial period may be set to "0 to 5 minutes".

At this time, the processor 110 may obtain a plurality of reaction images in the predetermined time unit for the predetermined initial period. For example, when the predetermined initial period is "0 to 5 minutes" and the predetermined time unit is "10 seconds", the processor 110 may obtain 30 (= 6 X 5) reaction images.

The analysis result prediction model includes a convolution neural network (CNN), a long short-term memory (LSTM), and a generative adversarial network (GAN), and details thereof will be described below.

That is, as illustrated in FIG. 3, a change in color intensity over time may be predicted through a reaction image for interaction of the optical based kit including one test-line and one control-line and a sample at a specific timing. Accordingly, as illustrated in FIG. 4, a concentration for a predetermined result time (for example, 15 minutes) may be predicted through the reaction image for the predetermined initial period.

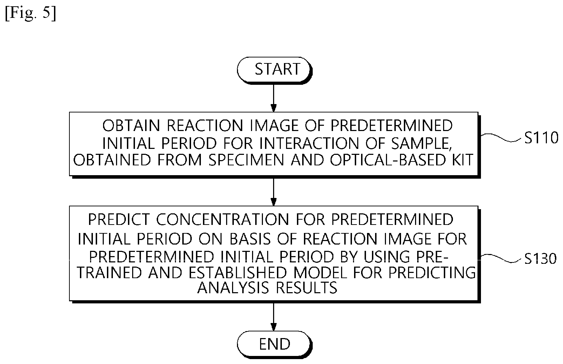

Now, a deep learning based analysis result predicting method according to the present invention will be described with reference to FIG. 5.

FIG. 5 is a flowchart for explaining a deep learning-based analysis result predicting method according to a preferred embodiment of the present invention.

Referring to FIG. 5, the processor 110 of the analysis result predicting device 100 obtain a reaction image of a predetermined initial period for an interaction of a sample obtained from a specimen and an optical based kit (S 110).

At this time, the processor 110 may obtain a plurality of reaction images in the predetermined time unit for the predetermined initial period.

The processor 110 performs a pre-processing process of the reaction image before inputting the reaction image to the analysis result prediction model.

That is, when the optical-based kit includes a test-line and a control-line, the processor 110 obtains a reaction image of an area corresponding to the test-line. Here, the size of the area may be set in advance to be "200X412 size".

In the meantime, when the optical-based kit includes a plurality of test-lines, the processor 110 may obtain a reaction image of an area corresponding to one or more predetermined test-lines, among the plurality of test-lines.

At this time, the processor 100 may obtain a reaction image including all the areas corresponding to one or more predetermined test-lines, among the plurality of test-lines or obtain a reaction image for every test-line to distinguish the areas corresponding to one or more predetermined test-lines, among the plurality of test-lines, for every test-line.

Thereafter, the processor 110 predicts a concentration for the predetermined result time on the basis of the reaction image for the predetermined initial period, using the pre-trained and established analysis result prediction model (S130).

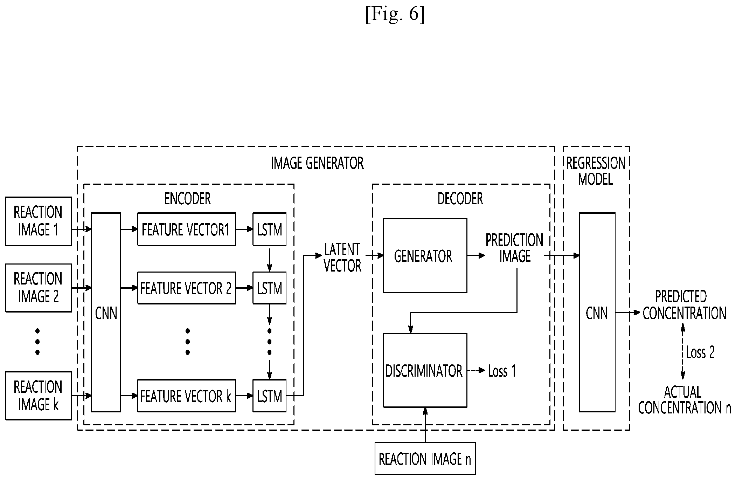

Now, a structure of the analysis result prediction model according to a preferred embodiment of the present invention will be described with reference to FIGS. 6 to 8.

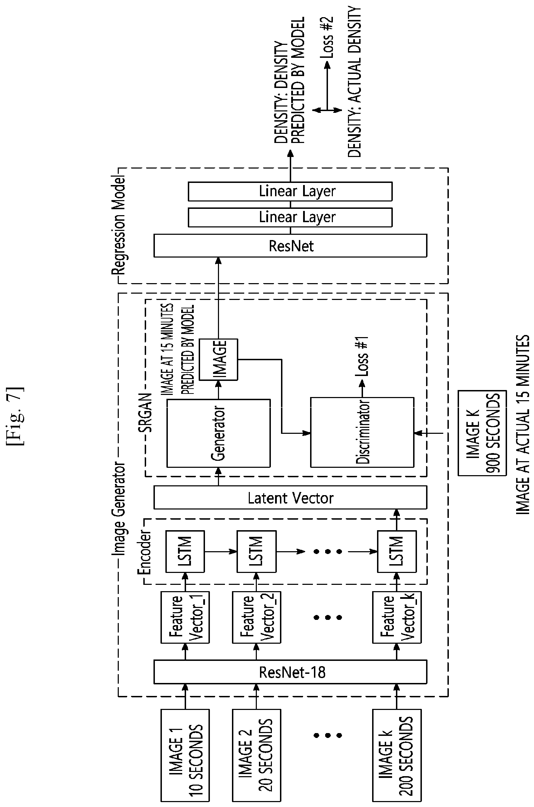

FIG. 6 is a view for explaining an example of a structure of an analysis result prediction model according to a preferred embodiment of the present invention and FIG. 7 is a view for explaining an implementation example of an analysis result prediction model illustrated in FIG. 6.

Referring to FIG. 6, an example of the analysis result prediction model according to the present invention includes an image generator and a regression model.

The image generator includes a convolution neural network (CNN), a long short-term memory (LSTM), and a generative adversarial network (GAN), generates a prediction image corresponding to a predetermined result time on the basis of a plurality of input reaction images, and outputs the generated prediction image.

To this end, the image generator includes an encoder and a decoder.

The encoder obtains a feature vector from each of the plurality of input reaction images using the convolution neural network (CNN), obtains a latent vector on the basis of the plurality of obtained feature vectors using the long short term memory (LSTM), and outputs the obtained latent vector. That is, the encoder calculates a relationship of a concentration, a variance of color intensity, and a time from the plurality of feature vectors to generate the latent vector.

The decoder generates a prediction image using the generative adversarial network (GAN) on the basis of the latent vector obtained from the encoder, and outputs the generated prediction image.

That is, the decoder includes a generator which generates the prediction image on the basis of the latent vector and outputs the generated prediction image and a discriminator which compares a prediction image generated by the generator and an actual image corresponding to the predetermined result time of the learning data and outputs a comparison result.

At this time, the decoder is trained using the learning data so as to discriminate that the prediction image obtained based on the latent vector is an actual image.

A regression model includes a convolution neural network (CNN) and outputs a predicted concentration for a predetermined result time on the basis of the prediction image generated by the image generator. That is, the regression model obtains a feature vector of the prediction image and obtains a predicted concentration by causing the obtained feature vector to pass through two linear layers.

At this time, the regression model is trained using the learning data to minimize the difference of the predicted concentration for the predetermined result time obtained on the basis of the reaction image of the learning data and the actual concentration for the predetermined result time of the learning data.

In the meantime, the discriminator is a module required for a learning process of the analysis result prediction model and is removed from the analysis result prediction model after completing the learning.

For example, as illustrated in FIG. 7, when the plurality of reaction images (images 1 to k of FIG. 7) obtained at every 10 seconds for the initial period (10 to 200 seconds) is input to the analysis result prediction model, the analysis result prediction model obtains feature vectors (Feature Vector_1 to Feature Vector_k) using ResNet-18 which is a convolution neural network) from the plurality of reaction images (images 1 to k of FIG. 7). Thereafter, the analysis result prediction model inputs the plurality of obtained feature vectors (Feature Vector_1 to Feature Vector_k of FIG. 7) to a corresponding long short term memory (LSTM).

Thereafter, the analysis result prediction model inputs the latent vector output from the long short term memory (LSTM) to a generator of "SRGAN" which is a generative adversarial neural network (GAN). By doing this, the analysis result prediction model generates a prediction image corresponding to the result time (15 minutes) on the basis of the latent vector. Thereafter, the analysis result prediction model inputs the generated prediction image to discriminators of ResNet and SRGAN which are the convolution neural networks (CNN). Here, the discriminator compares the prediction image and the actual image corresponding to the result time (15 minutes) and provides the comparison result to the generator.

By doing this, the analysis result prediction model outputs a predicted concentration for the result time (15 minutes).

Here, the analysis result prediction model may be trained using the learning data to minimize two losses. A first loss (Loss #1 in FIG. 7) is to discriminate the prediction image obtained on the basis of the latent vector as an actual image. A second loss (Loss #2 in FIG. 7) is to minimize the difference between the predicted concentration for the result time (15 minutes) and the actual concentration for the result time (15 minutes).

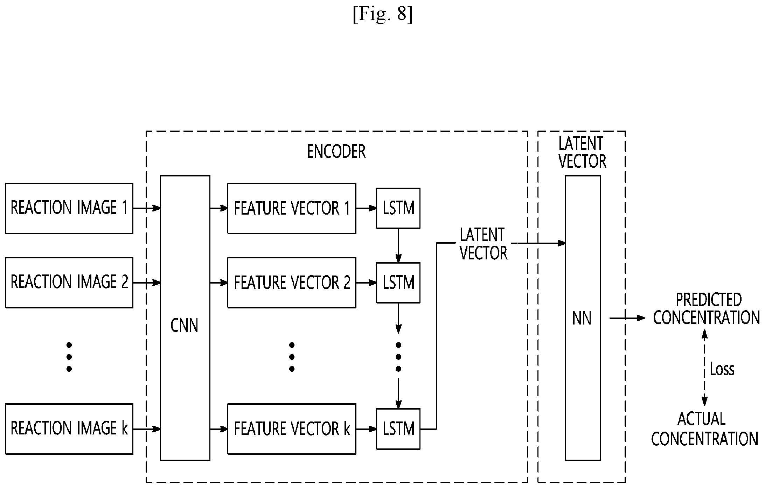

FIG. 8 is a view for explaining another example of a structure of an analysis result prediction model according to a preferred embodiment of the present invention.

Referring to FIG. 8, another example of the analysis result prediction model according to the present invention is substantially the same as an example (see FIG. 6) of the analysis result prediction model which has been described above and a process of generating an image is omitted from the example (see FIG. 6) of the analysis result prediction model.

That is, another example of the analysis result prediction model removes a decoder from the example (see FIG. 6) of the analysis result prediction model to include an encoder and a regression model.

The encoder includes a convolution neural network (CNN) and a long short term memory (LSTM) and generates a latent vector on the basis of the plurality of input reaction images and outputs the generated latent vector. In other words, the encoder obtains a feature vector from each of the plurality of input reaction images using the convolution neural network (CNN), obtains a latent vector on the basis of the plurality of obtained feature vectors using the long short term memory (LSTM), and outputs the obtained latent vector.

The regression model includes a neural network (NN) and outputs a predicted concentration for a predetermined result time on the basis of the latent vector obtained through the encoder. At this time, the regression model is trained using the learning data to minimize the difference of the predicted concentration for the predetermined result time obtained on the basis of the reaction image of the learning data and the actual concentration for the predetermined result time of the learning data.

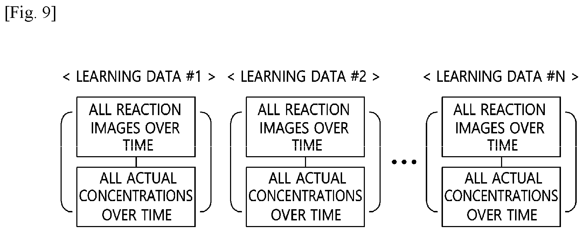

Now, learning data used for a training process of the analysis result prediction model according to a preferred embodiment of the present invention will be described with reference to FIGS. 9 to 12.

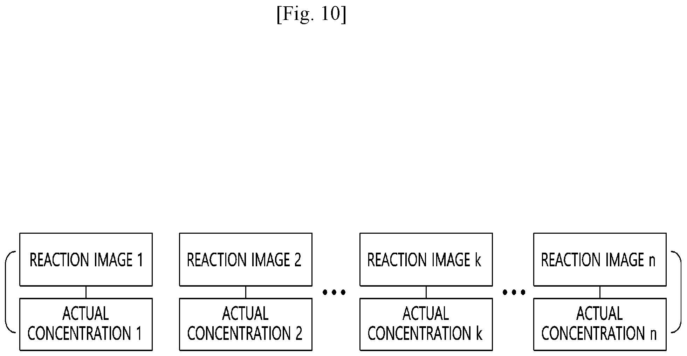

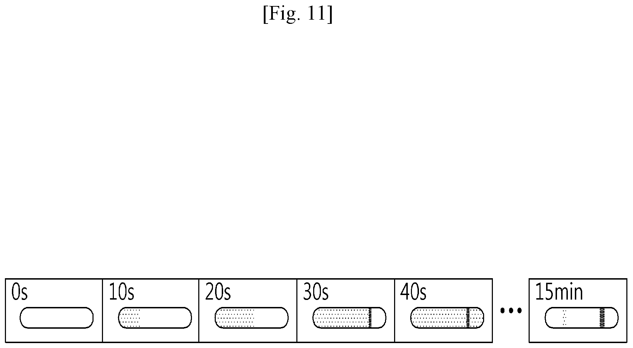

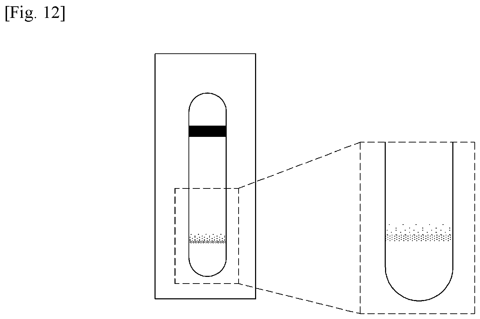

FIG. 9 is a view for explaining learning data used for a learning process of an analysis result prediction model according to a preferred embodiment of the present invention, FIG. 10 is a view for explaining a configuration of learning data illustrated in FIG. 9, FIG. 11 is a view for explaining an example of a reaction image illustrated in FIG. 10, and FIG. 12 is a view for explaining an example of a pre-processing process of a reaction model according to a preferred embodiment of the present invention.

The learning data used for the learning process of the analysis result prediction model according to the present invention may be configured by a plurality of data as illustrated in FIG. 9.

That is, as illustrated in FIG. 10, each learning data includes all reaction images (reaction image 1 to reaction image n in FIG. 10) over time and all actual concentrations (actual concentration 1 to actual concentration (actual concentration 1 to actual concentration n of FIG. 10) over time. Here, "Reaction Image 1 to Reaction image k" illustrated in FIG. 10 indicate reaction images of the initial period. For example, as illustrated in FIG. 11, the reaction image is obtained at every 10 minutes from a reaction start time (0 minute) to the result time (15 minutes).

At this time, some of all reaction images (reaction image 1 to reaction image n in FIG. 10) over time and all actual concentrations (actual concentration 1 to actual concentration n in FIG. 10) over time are used as learning data and the remaining is used as test data and validation data. For example, odd-numbered reaction images and actual concentrations are used as training data to train the analysis result prediction model. Even-numbered reaction images and actual concentrations are used as test data and validation data to test and validate the analysis result prediction model.

Further, a pre-processing process of the reaction image is performed before inputting the reaction image to the analysis result prediction model. For example, as illustrated in FIG. 12, a reaction image of an area corresponding to the test-line is obtained from the entire images.

Now, the implementation example of the deep learning-based analysis result predicting device 100 according to a preferred embodiment of the present invention will be described.

First, a reaction image of an initial period (for example, "0 minute to 5 minutes" for interaction of a sample obtained from the specimen and an optical-based kit (for example, a lateral flow assay kit) is obtained from an imaging device (not illustrated). At this time, the camera photographs in a predetermined time unit (for example, "10 seconds") to obtain a plurality of reaction images. The camera may capture a video during an initial period and extract an image frame from the captured video in a predetermined time unit to obtain a plurality of reaction images.

Thereafter, the camera provides the reaction image to the analysis result predicting device 100 according to the present invention directly or via an external server, through wireless/wired communication. When the analysis result predicting device 100 according to the present invention includes a capturing module, the analysis result predicting device may directly obtain the reaction image.

The analysis result predicting device 100 according to the present invention predicts a concentration for the result time (for example, "15 minutes" using the previously stored analysis result prediction model on the basis of the reaction image of the initial period and outputs the result. The analysis result predicting device 100 according to the present invention also provides the reaction image of the initial period to an external server in which the analysis result prediction model is stored through the wireless/wired communication and receives the predicted concentration for the result time from the external server to output the result.

The operation according to the embodiment of the present disclosure may be implemented as a program instruction which may be executed by various computers to be recorded in a computer readable storage medium. The computer readable storage medium indicates an arbitrary medium which participates to provide a command to a processor for execution. The computer readable storage medium may include solely a program command, a data file, and a data structure or a combination thereof. For example, the computer readable medium may include a magnetic medium, an optical recording medium, and a memory. The computer program may be distributed on a networked computer system so that the computer readable code may be stored and executed in a distributed manner. Functional programs, codes, and code segments for implementing the present embodiment may be easily inferred by programmers in the art to which this embodiment belongs.

The present embodiments are provided to explain the technical spirit of the present embodiment and the scope of the technical spirit of the present embodiment is not limited by these embodiments. The protection scope of the present embodiments should be interpreted based on the following appended claims and it should be appreciated that all technical spirits included within a range equivalent thereto are included in the protection scope of the present embodiments.

### [Explanation of Reference Numerals and Symbols]

100: Analysis result predicting device
110: Processor
130: Computer readable storage medium
131: Program
150: Communication bus
170: Input/output interface
190: Communication interface

## Claims

1. A deep learning-based analysis result predicting method, comprising:
a step of obtaining a reaction image for a predetermined initial period for an interaction of a sample obtained from a specimen and an optical-based kit; and
a step of predicting a concentration for a predetermined result time on the basis of the reaction image for the predetermined initial period, using a pre-trained and established analysis result prediction model.

2. The deep learning-based analysis result predicting method of claim 1, wherein the step of obtaining a reaction image is configured by obtaining a plurality of reaction images in a predetermined time unit for the predetermined initial period.

3. The deep learning-based analysis result predicting method of claim 2, wherein the analysis result prediction model includes:
an image generator includes a convolution neural network (CNN), long short-term memory (LSTM), and a generative adversarial network (GAN), and generates a prediction image corresponding to a predetermined result time on the basis of an input reaction image, and outputs the generated prediction image; and
a regression model which includes the convolution neural network (CNN) and outputs a predicted concentration for a predetermined result time on the basis of the prediction image generated by the image generator, and
the regression model is trained using the learning data to minimize the difference of the predicted concentration for the predetermined result time obtained on the basis of the reaction image of the learning data and the actual concentration for the predetermined result time of the learning data.

4. The deep learning-based analysis result predicting method of claim 3, wherein the image generator includes:
an encoder which obtains a feature vector from the input reaction image using the convolution neural network (CNN), obtains a latent vector on the basis of the obtained feature vector using the long short term memory (LSTM), and outputs the obtained latent vector; and
a decoder which generates the prediction image on the basis of the latent vector obtained from the encoder using the generative adversarial network (GAN), and outputs the generated prediction image.

5. The deep learning-based analysis result predicting method of claim 4, wherein the decoder includes:
a generator which generates the prediction image on the basis of the latent vector and outputs the generated prediction image; and
a discriminator which compares the prediction image generated by the generator and an actual image corresponding to a predetermined result time of the learning data and outputs a comparison result, and
it is trained to discriminate that the prediction image obtained on the basis of the latent vector is the actual image using the learning data.

6. The deep learning-based analysis result predicting method of claim 1, wherein the step of obtaining a reaction image is configured by obtaining the reaction image of an area corresponding to the test-line when the optical-based kit includes a test-line and a control-line.

7. The deep learning-based analysis result predicting method of claim 6, wherein the step of obtaining a reaction image is configured by obtaining the reaction image of the area corresponding to one or more predetermined test-lines, among a plurality of test-lines when the optical-based kit includes a plurality of test-lines.

8. The deep learning-based analysis result predicting method of claim 7, wherein the step of obtaining a reaction image is configured by obtaining the reaction image including all areas corresponding to one or more predetermined test-lines, among the plurality of test-lines or obtaining the reaction image for every test-line to distinguish areas corresponding to one or more predetermined test-lines, among the plurality of test-lines for every test-line.

9. A computer program stored in a computer readable storage medium to allow a computer to execute the deep learning-based analysis result predicting method according to Claims 1.

10. A deep learning-based analysis result predicting device which predicts an analysis result based on deep learning, comprising:
a memory which stores one or more programs to predict an analysis result; and
one or more processors which perform an operation for predicting the analysis result according to one or more programs stored in the memory,
wherein the processor predicts a concentration for a predetermined result time on the basis of a reaction image of a predetermined initial period for an interaction of a sample obtained from a specimen and an optical-based kit, using a pre-trained and established analysis result prediction model.

11. The deep learning-based analysis result predicting device of claim 10, wherein the processor obtains a plurality of reaction images in a predetermined time unit for the predetermined initial period.

12. The deep learning-based analysis result predicting device of claim 11, wherein the analysis result prediction model includes:
an image generator includes a convolution neural network (CNN), long short-term memory (LSTM), and a generative adversarial network (GAN), and generates a prediction image corresponding to a predetermined result time on the basis of an input reaction image, and outputs the generated prediction image; and
a regression model which includes the convolution neural network (CNN) and outputs a predicted concentration for a predetermined result time on the basis of the prediction image generated by the image generator, and
the regression model is trained using the learning data to minimize the difference of the predicted concentration for the predetermined result time obtained on the basis of the reaction image of the learning data and the actual concentration for the predetermined result time of the learning data.

13. The deep learning-based analysis result predicting device of claim 12, wherein the image generator includes:
an encoder which obtains a feature vector from the input reaction image using the convolution neural network (CNN), obtains a latent vector on the basis of the obtained feature vector using the long short term memory (LSTM), and outputs the obtained latent vector; and
a decoder which generates the prediction image on the basis of the latent vector obtained from the encoder using the generative adversarial network (GAN), and outputs the generated prediction image.
